# EUROPEAN PATENT APPLICATION

(11) **EP 3 995 165 A2**
(43) Date of publication of application: **11.05.2022**
(21) Application number: 20847943.6
(22) Date of filing: 24.07.2020
(51) Int. Cl.: A61M 5/24, A61M 5/28, A61M 5/34, A61M 5/31

(54) **DRUG INJECTION CARTRIDGE AND DRUG INJECTOR COMPRISING SAME**

(30) Priority: 26.07.2019 KR 20190090565
(71) Applicant: Noh, Wook Rea, Suwon-si, Gyeonggi-do 16305 (KR)
(72) Inventor: Noh, Wook Rea, Suwon-si, Gyeonggi-do 16305 (KR)
(74) Representative: Isarpatent
(86) International application number: PCT/KR2020/009746
(87) International publication number: WO 2021/020811

(57) **Abstract**

The present invention relates to a drug injection cartridge and a drug injector comprising same, and has an anti-pushing part (100) formed between a needle part (10) and a cartridge (20), thereby preventing a rubber film from being expanded by pressure applied to the cartridge (20), such that a drug does not leak, and the accuracy of drug injection can be improved.

## Description

### TECHNICAL FIELD

The present disclosure relates to a drug injection cartridge and a drug injector including the same and, more particularly, a drug injection cartridge having an anti-slip portion between a needle and the cartridge to prevent a rubber film provided on one side of the cartridge from being expanded by pressure when a drug is being injected, thereby preventing the drug from leaking, and a drug injector including the same.

### BACKGROUND ART

Drug injectors are self-contained devices by which a user requiring self-management while having no formal medical training may inject a drug (or a medicine) into his or her own body.

In general, the drug injector generally includes a needle (i.e., a pen needle) through which a drug is injected into a human body, a cartridge containing the drug, a housing accommodating the cartridge and allowing a plunger to be introduced thereinto, and the plunger configured to be introduced into the housing to eject the drug.

In addition, drug injectors are categorized as a pump type drug injector attachable to a garment or a belt and a pen type drug injector which may be carried in a garment pouch, a bag, or the like

The pump type drug injector is attached to a user, with a needle of the drug injector being inserted into the hypodermis of the user, so as not to restrict the activities of the user. The user may freely use the pump type drug injector several times or continuously by adjusting the dose of a drug.

The pen type drug injector may be operated in a simple manner. The pen type drug injector is mounted with a cartridge containing a drug to be used by replacing a needle. It is possible to inject a drug in a simple manner using the pen type drug injector when the user moves frequently.

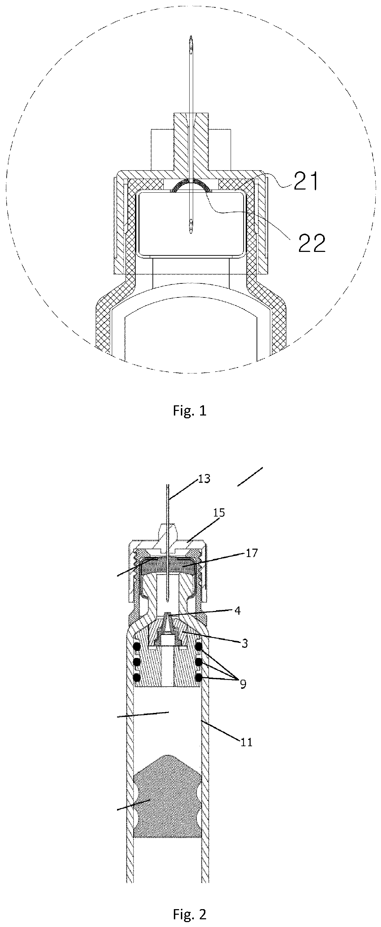

In a drug injector including a cartridge distributed in the market, a disposable needle is introduced into and fastened to a rubber film provided on one side of the cartridge, as illustrated in FIG. 1.

However, since a hollow space is defined between the cartridge and the disposable needle, the rubber film of the cartridge may be expanded like a balloon by pressure applied during injection of a drug. As the contact portion between the disposable needle and the rubber film is weakened by the expansion, the drug may leak, which is problematic.

Background-art technologies for overcoming the above problem may include PCT Publication WO2015/177082 (hereinafter, referred to as Document 1), Korean Patent Application Publication No. 10-1996-0700677 (hereinafter, referred to as Document 2), Japanese Patent No. 5933541 (hereinafter, referred to as Document 3), and Korean Patent Application Publication No. 10-2014-0138668 (hereinafter, referred to as Document 4).

Document 1 relates to a medical cartridge including a one-way valve. The cartridge is configured such that the one-way valve restrains an outlet end from inside the cartridge but prevents a drug from flowing from the outlet end into the cartridge, thereby minimizing the drug from being contaminated.

In the cartridge of Document 1, as illustrated in FIG. 2, a hollow space is defined between the cartridge and a needle. A rubber film may be expanded like a balloon by pressure applied during injection of a drug, thereby causing the drug to leak. Thus, the accuracy of injection of the drug may be lowered, which is problematic.

Document 2 relates to a cartridge assembly for freeze-dried pharmaceuticals and a freeze-drying method, wherein the cartridge assembly forms a syringe part of a syringe device. The syringe device includes a cylindrical glass cartridge containing a drug, a sealing cap, a cartridge protective container, and a plunger.

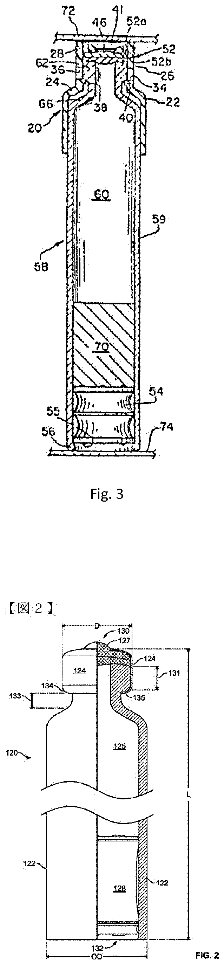

In the syringe device of Document 2, as illustrated in FIG. 3, an elastic seal is positioned between the cap and a first open area of the cartridge to form an air blocking layer for an inlet positioned below the elastic seal. However, when the syringe device is applied to a drug injector in which the cartridge itself is replaced, a drug may leak, which is problematic.

Document 3 relates to a drug cartridge having non-standard dimensions. In the drug cartridge, the diameter of a head portion of an open area has a non-standard dimension. A septum is provided on one side of the cartridge to prevent leakage during injection of a drug.

In the cartridge of Document 3, as illustrated in FIG. 4, the septum protrudes from one side of the cartridge. When the cartridge is coupled to a disposable needle, a hollow space may be defined. Thus, there is a problem in that a drug may be contaminated due to leakage or the accuracy of injection may be lowered.

Document 4 relates to an adaptor configured to be coupled to a medical container. The adaptor is configured to be coupled to a vial having a collar configured to be closed by a septum having an outer surface facing outside the vial.

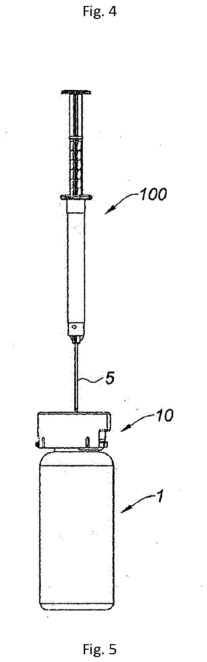

As illustrated in FIG. 5, when the adaptor of Document 4 is applied to one side of a cartridge, the adaptor is not consistent with drug injector standards, thereby making the use thereof impossible. The adaptor has a structure that cannot be coupled to a disposable needle.

### Related Art Document

(Document 1) PCT Publication WO2015/177082
(Document 2) Korean Patent Application Publication No. 10-1996-0700677
(Document 3) Japanese Patent No. 5933541
(Document 4) Korean Patent Application Publication No. 10-2014-0138668

### DISCLOSURE

### Technical Problem

Accordingly, the present disclosure has been made in consideration of the above-described problems occurring in the related art, and the present disclosure is intended to form an anti-slip portion on one side of a drug injection cartridge to prevent a rubber film provided on one side of the cartridge from being expanded by pressure, thereby preventing a drug from leaking.

The present disclosure is also intended to increase a contact portion between a rubber film provided on one side of the cartridge and a disposable needle, thereby improving the accuracy of injection of a drug.

The present disclosure is also intended to allow a user to selectively use a drug injector in which a cartridge used therein may be replaced or only a needle can be replaced.

### Technical Solution

In order to realize at least one of the above-described objectives, a drug injection cartridge according to the present disclosure may include a needle unit (10), a cartridge (20), and a cartridge housing (30), and further include an anti-slip portion (100) provided between the needle unit (10) and the cartridge (20) to prevent a rubber film provided on one side of the cartridge (20) from being expanded, thereby preventing a drug from leaking.

The anti-slip portion (100) may include a first anti-slip portion (110) or a second anti-slip portion (120).

The first anti-slip portion (110) according to the present disclosure may protrude from an inner portion of the needle unit (10) in contact with the rubber film (22) provided on one side of the cartridge (20). The second anti-slip portion (120) may be provided on one side of the cartridge housing (30) in contact with the rubber film (22) provided on one side of the cartridge (20) and protrudes toward a second space (33), and a third space (34) for coupling to the needle unit (10) may be provided.

The drug injection cartridge according to the present disclosure may be applied to a drug injector configured to inject a drug, such as insulin or hormone.

### Advantageous Effects

In the drug injection cartridge according to the present disclosure, the anti-slip portion may prevent the rubber film provided on one side of the cartridge from being expanded by pressure during injection of a drug to prevent the drug from leaking, thereby improving the accuracy of injection.

In addition, the drug injection cartridge according to the present disclosure may prevent a drug from being contaminated by preventing the drug from leaking.

Furthermore, the drug injection cartridge according to the present disclosure may be simply and selectively used depending on the shape of a drug injector of a user.

### DESCRIPTION OF DRAWINGS

FIG. 1 is an expanded view illustrating a typical drug injection cartridge;
FIGS. 2 to 5 illustrate technologies disclosed in background-art documents of the present disclosure;
FIG. 6 is a view illustrating an example of a drug injection cartridge according to the present disclosure;
FIG. 7 is an exploded view of the drug injection cartridge according to the present disclosure;
FIG. 8 is a cross-sectional view of a needle unit according to the present disclosure;
FIG. 9 is an exploded view of the drug injection cartridge according to the present disclosure;
FIG. 10 is a view illustrating an example of a drug injection cartridge according to another embodiment of the present disclosure;
FIG. 11 is an exploded view of the drug injection cartridge according to another embodiment of the present disclosure;
FIG. 12 is a cross-sectional view of the cartridge housing according to the present disclosure; and
FIG. 13 is an exploded view of the drug injection cartridge according to another embodiment of the present disclosure.

### <Description of Reference Numerals of Drawings>

10: needle unit; 11: needle; 12: first body; 13: first space; 14: first support;
20: cartridge; 21: first protective portion; 22: rubber film;
30: cartridge housing; 31: second body; 32: second protective portion; 33: second space; 34: third space; and
100: anti-slip portion; 110: first anti-slip portion; 120: second anti-slip portion

### BEST MODE

Hereinafter, embodiments of the present disclosure will be described in detail.

Objectives, features, and advantages of the present disclosure will be apparent from the following embodiments.

The present disclosure should not be construed as being limited to the embodiments to be disclosed herein and may be embodied in a variety of different forms. The following embodiments will be provided for illustrative purposes to fully convey the concept of the present disclosure to those having ordinary knowledge in the technical field, and should be interpreted as including all modifications, equivalents, and substitutions without departing from the technical idea and scope of the present disclosure.

Therefore, the present disclosure is not limited by the following embodiments but should be interpreted as including all modifications without departing from the technical idea and scope of the present disclosure. That is, those having ordinary knowledge in the technical field to which the present disclosure pertains can make various modifications and variations of the present disclosure by adding, changing, deleting, or supplementing elements without departing from the principle of the present disclosure described in the Claims, and such modifications and variations should be included within the scope of the present disclosure.

Although the present disclosure may be modified variously and have a variety of embodiments, specific embodiments will be illustrated in the drawings and described in detail. In the drawings, the sizes of the elements and the relative sizes of the elements may be exaggerated for a better understanding of the present disclosure. In addition, the shapes of the elements illustrated in the drawings may be changed more or less due to changes in fabrication process or the like.

Therefore, the following embodiments should not be limited to the shapes illustrated in the drawings unless expressly so stated herein but should be understood as including some variations.

In the meantime, several embodiments of the present disclosure may be coupled to other embodiments unless explicitly stated to the contrary. In particular, any preferable or advantageous features may be combined with any other feature or features. That is, various aspects, features, embodiments, or implementations of the present disclosure may be used either alone or in various combinations.

It should be understood that each of terms used herein is to describe a specific embodiment but is not to be limited by the Claims. Unless otherwise specified, all terms including technical and scientific terms used herein have the same meaning as that commonly understood by those having ordinary knowledge in the art. As used herein, singular forms are intended to include plural forms unless the context clearly indicates otherwise.

In the description of the present disclosure, detailed descriptions of well-known functions and components incorporated herein will be omitted when it is determined that the description may make the subject matter of the present disclosure rather unclear.

The term "drug" as used herein may include at least one selected from, but is not limited to, insulin, hormone, low-molecular-weight heparin, or derivatives thereof.

A drug injection cartridge according to the present disclosure may include a needle unit 10, a cartridge 20, and a cartridge housing 30.

The needle unit 10 may be a disposable pen needle including a needle 11, a first body 12, a first space 13, and a first support 14.

In addition, the needle unit 10, except for the needle 11, may be formed of a synthetic resin, particularly, polyethylene (PE) or polypropylene (PP), but is not limited thereto.

The needle 11 is inserted into and received in the first support 14. The outer diameter of the needle 11 may be in the range from 0.2 mm to 1.7 mm, but is not limited thereto. The outer diameter may vary depending on the intended use.

When the outer diameter of the needle 11 is less than 0.2 mm, it is difficult to inject a drug into a patient using the needle unit although the patient may feel less pain. In particular, when the drug has higher viscosity, it is more difficult to inject a drug.

In addition, when the needle 11 is formed slender, the needle 11 is easily breakable, which is problematic.

When the outer diameter of the needle 11 is greater than 1.7 mm, pain caused to the patient may be significantly increased, which is problematic.

Thus, the outer diameter of the needle 11 may be categorized according to the intended use.

That is, when the needle 11 is intended to be used for intravenous injection, the outer diameter of the needle 11 may be in the range from 0.56 mm to 1.6 mm. When the needle 11 is intended to be used for intramuscular injection, the outer diameter of the needle 11 may be in the range from 0.6 mm to 0.86 mm.

In addition, when the needle 11 is intended to be used for subcutaneous injection, the outer diameter of the needle 11 may be in the range from 0.25 mm to 0.5 mm. When the needle 11 is intended to be used for intradermal injection, the outer diameter of the needle 11 may be in the range from 0.4 mm to 0.5 mm.

The first body 12 may have the shape of a cylinder, with the other end thereof being open.

In addition, the first body 12 has the first space 13 such that one end of the cartridge housing 30 including the cartridge 20 may be introduced into the first body 12.

The inner diameter of the first space 13 may be the same as the outer diameter of one end of the cartridge housing 30.

The first support 14 protrudes from the central portion of one end of the first body 12. A hole may be formed in the central portion of the first support 14 to support and fix the needle 11 introduced into the first body 12.

Here, the needle 11 may be introduced into and fixed by the hole formed in the central portion of the first support 14.

The diameter of the hole formed in the central portion of the first support 14 may be the same as the diameter of the needle 11 and thus is not limited to a specific numerical value.

The cartridge 20 may include a container in which a drug to be injected into a user is contained, a first protective portion 21, and a rubber film 22.

The container has the shape of a cylinder to protect the drug. The container may include the first protective portion 21 and the rubber film 22 provided on one end and a plunger (not shown) provided on the other end.

In addition, the container is formed of glass or a synthetic resin to protect the drug. When the container is formed of a synthetic resin, polyvinyl chloride (PVC), polyethylene (PE), or polypropylene (PP) may be used, but the present disclosure is not limited thereto.

Particularly, the container may be formed of glass, PE, or PP.

Glass is advantageous in terms of thermal stability, transparency, and resistance to rapid temperature changes.

PE can block the movement of oxygen to prevent the drug from being exposed to oxygen, thereby preventing the drug from being altered. PP is advantageous in terms of harmlessness to the human body and, when compared to PE, in terms of transparency, tensile strength, heat resistance, and chemical resistance.

The first protective portion 21 is provided on one end of the container, and is configured to prevent the rubber film 22 from being released from the container.

The rubber film 22 is provided on one end of the container, and is configured to be fixed to the container by the first protective portion 21.

Here, when the needle unit 10 is introduced through the rubber film 22 in the direction of the cartridge 20, the needle 11 may penetrate the rubber film 22, so that the drug may leak through the needle 11.

The cartridge housing 30 protects the cartridge 20. The cartridge housing 30 may include a second body 31, a second protective portion 32, a second space 33, and a third space 34 provided on one end to be coupled to the needle unit 10.

In addition, the cartridge housing 30 may be formed of a synthetic resin, particularly, PE or PP, but is not limited thereto.

The second body 31 has the shape of a cylinder. The second protective portion 32 protrudes from one end of the second body 31. The other end of the second body 31 has a hole through which the cartridge 20 can be introduced into the second body 31. The second space 33 is configured to accommodate the cartridge 20 introduced thereinto.

The second protective portion 32 is a portion to which the needle unit 10 is coupled, and the third space 34 may be defined in the central portion of the second protective portion 32 such that the needle 11 may penetrate the central portion of the second protective portion 32.

In addition, the second protective portion 32 may be provided with a thread or a holding portion (not shown) to enhance safety in use after the needle unit 10 is coupled thereto. Due to this configuration, the needle unit 10 can be prevented from being separated from the cartridge housing 30 during injection of a drug.

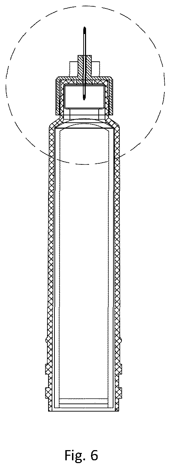

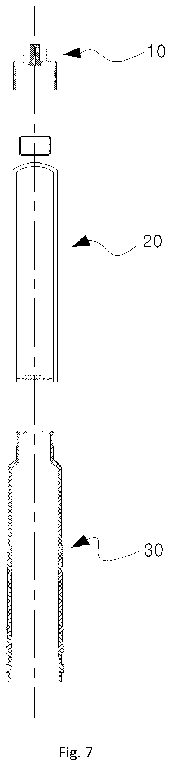

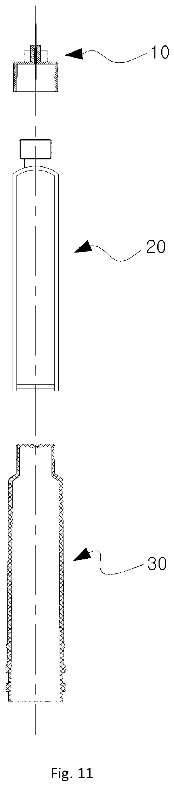

As illustrated in FIGS. 6 to 13, the drug injection cartridge may have an anti-slip portion 100 between the needle unit 10 and the cartridge 20. Here, the anti-slip portion 100 may prevent the rubber film 22 provided on one side of the cartridge 20 from being expanded like a balloon by pressure applied to the drug injection cartridge as in the case of FIG. 1, thereby preventing the drug from leaking.

The anti-slip portion 100 may include a first anti-slip portion 110 or a second anti-slip portion 120.

### <First Embodiment>

The drug injection cartridge may include the first anti-slip portion 110.

Features except for the first anti-slip portion 110 are the same as those described above, and thus descriptions thereof will be omitted.

As illustrated in FIGS. 6 to 9, the first anti-slip portion 110 may protrude from an inner portion of the needle unit 10 to be in contact with the surface of the rubber film 22 provided on one side of the cartridge 20.

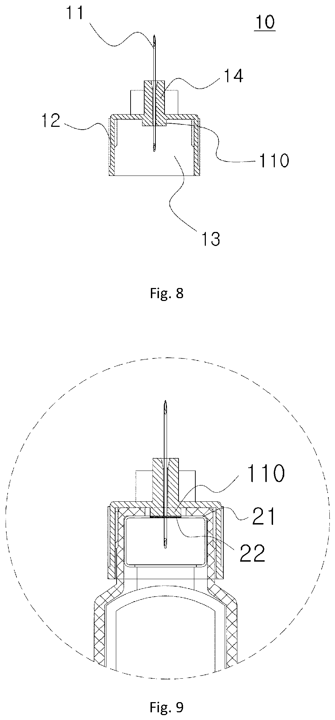

As illustrated in FIG. 8, the first anti-slip portion 110 protrudes in the direction of the first space 13 inside the needle unit 10. The hole provided in the central portion of the first support 14 may extend through the first anti-slip portion 110 such that the needle 11 may penetrate the first anti-slip portion 110.

The first anti-slip portion 110 may be formed of the same material as the needle unit 10, except for the needle 11. Particularly, the first anti-slip portion 110 may be formed of PE or PP.

In addition, as illustrated in FIG. 9, the length of the first anti-slip portion 110 may be the same as or greater than the length of the rubber film 22. During use of the cartridge 20, the first anti-slip portion 110 may prevent the rubber film 22 from being expanded like a balloon by pressure as in the case of FIG. 1, thereby preventing the drug from leaking.

In particular, when the phenomenon as in the case of FIG. 1 occurs in a situation in which a greater dose of drug is injected, a greater portion of drug may leak, thereby lowering the accuracy of injection of the drug.

In contrast, according to the present disclosure, even when pressure is applied, the rubber film 22 may remain flat or planar to prevent the drug from leaking, thereby advantageously improving the accuracy of injection of the drug.

In addition, the contact portion between the first anti-slip portion 110 and the rubber film 22 is not weakened. Thus, there is an advantage in that, even when pressure is additionally applied, the drug does not leak.

Furthermore, since the first anti-slip portion 110 is provided on the needle unit 10, the cartridge may be used only once to prevent the drug from being contaminated. In this manner, the cartridge may be coupled to a manual drug injector for use thereof.

For example, the manual drug injector may be a manual pen type drug injector but is not limited thereto.

The manual drug injector may be a drug injector fabricated by a well-known method.

### MODE FOR INVENTION

### <Second Embodiment>

The drug injection cartridge may include a second anti-slip portion 120.

Features except for the second anti-slip portion 120 are the same as those described above, and thus descriptions thereof will be omitted.

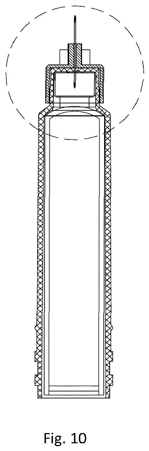

As illustrated in FIGS. 10 to 13, the second anti-slip portion 120 may be provided on one side of the cartridge housing 30 to be in contact with the surface of the rubber film 22 provided on one side of the cartridge 20.

The second anti-slip portion 120 may be formed of the same material as the cartridge housing 30. Particularly, the second anti-slip portion 120 may be formed of PE or PP.

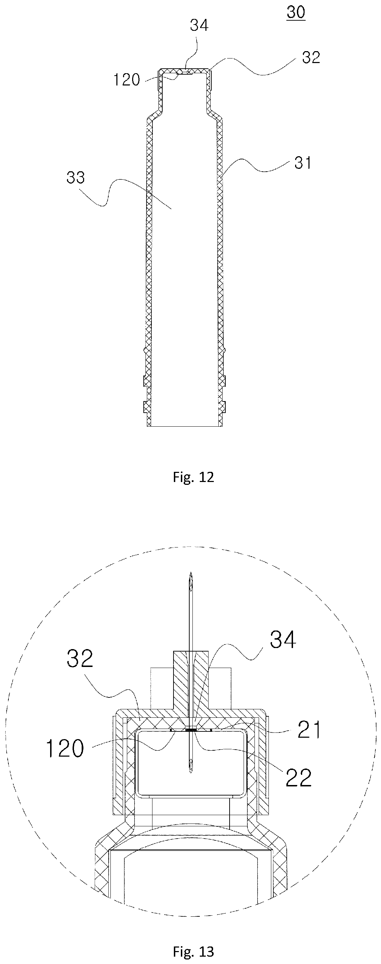

As illustrated in FIG. 12, the second anti-slip portion 120 may be formed by longitudinally extending the second protective portion 32 provided on one side of the cartridge housing 30. The second anti-slip portion 120 may protrude by forming a stepped portion in the direction of the second space 33.

In addition, since the second anti-slip portion 120 protrudes from an inner portion of the second protective portion 32, the extension of the third space 34 provided on the central portion of the second protective portion 32 may correspond to the second anti-slip portion 120 provided to be coupled to the needle unit 10.

The inner diameter of the third space 34 may be the same as the outer diameter of the needle 11 used in the needle unit 10 but is not limited thereto. This is because, when the inner diameter of the third space 34 is greater than the outer diameter of the needle 11, the second anti-slip portion 120 and the surface of the rubber film 22 may not be in close contact with each other, thereby forming a gap, through which a drug may leak.

That is, the inner diameter of the third space 34 may be the same as the outer diameter of the needle 11 in order to prevent the gap between the second anti-slip portion 120 and the surface of the rubber film 22.

In particular, as illustrated in FIG. 13, the second anti-slip portion 120 may be provided on the cartridge housing 30 to prevent the rubber film 22 of the cartridge 20 from being expanded by pressure as in the case of FIG. 1, thereby preventing the drug from leaking.

In addition, when the phenomenon as in the case of FIG. 1 occurs in a situation in which a greater dose of drug is injected, a greater portion of drug may leak, thereby lowering the accuracy of injection of the drug.

In contrast, according to the present disclosure, even when pressure is applied, the rubber film 22 may be maintained to be flat or planar by the second anti-slip portion 120, thereby advantageously preventing the drug from leaking.

In addition, the contact portion between the second anti-slip portion 120 and the rubber film 22 is not weakened. Thus, there is an advantage in that, even when pressure is additionally applied, the drug may not leak.

Furthermore, since the second anti-slip portion 120 is provided on the cartridge housing 30, only the cartridge 20 included inside the cartridge housing 30 may be replaced. Accordingly, it is possible to use the cartridge by coupling the cartridge to an automatic drug injector, i.e., a pump type drug injector or an automatic pen type drug injector.

The pump type drug injector or the automatic pen type drug injector may be a drug injector well-known in the art.

In a method in which only the needle unit 10 is replaced, the cartridge 20 is used until the drug contained therein is exhausted. Thus, the hole formed in the cartridge may be widened as needles are used. In order to prevent or reduce the problem in that the contact portion of the rubber film 22 is deteriorated as the hole is gradually widened by needles, the second anti-slip portion 120 may be provided on one side of the cartridge housing 30, thereby advantageously preventing deterioration in the performance of the rubber film 22.

### INDUSTRIAL APPLICABILITY

The present disclosure relates to a drug injection cartridge and a drug injector including the same. An anti-slip portion is provided between a needle and the cartridge to prevent a rubber film provided on one side of the cartridge from being expanded by pressure, thereby preventing a drug from leaking. Accordingly, the present disclosure is industrially applicable.

## Claims

1. A drug injection cartridge comprising a needle unit (10), a cartridge (20), and a cartridge housing (30),
the drug injection cartridge further comprising an anti-slip portion (100) provided between the needle unit (10) and the cartridge (20) to prevent a rubber film provided on one side of the cartridge (20) from being expanded, thereby preventing a drug from leaking.

2. The drug injection cartridge of claim 1, wherein the anti-slip portion (100) comprises a first anti-slip portion (110) or a second anti-slip portion (120).

3. The drug injection cartridge of claim 2, wherein the first anti-slip portion (110) protrudes from an inner portion of the needle unit (10) in contact with the rubber film (22) provided on one side of the cartridge (20).

4. The drug injection cartridge of claim 2, wherein the second anti-slip portion (120) is provided on one side of the cartridge housing (30) in contact with the rubber film (22) provided on one side of the cartridge (20) and protrudes toward a second space (33), and a third space (34) for coupling to the needle unit (10) is provided.

5. A drug injector comprising the drug injection cartridge of any one of claims 1 to 4.
